# EUROPEAN PATENT APPLICATION

(11) **EP 2 762 174 A1**
(43) Date of publication of application: **06.08.2014**
(21) Application number: 11872972.2
(22) Date of filing: 11.10.2011
(51) Int. Cl.: A61L 27/38, A61F 2/28, C12N 5/071

(54) **COMPOSITION FOR BONE REGENERATION**

(30) Priority: 29.09.2011 KR 20110099025
(71) Applicant: CG Bio Co., Ltd., Kyunggi-do 462-120 (KR)
(72) Inventor: SONG, Seok-Beom, Seongnam Gyeonggi-do 462-242 (KR); KIM, Soo-Yong, Goyang Gyeonggi-do 410-759 (KR); SO, Jung-Won, Seongnam Gyeonggi-do 463-500 (KR); SEO, Han-Sol, Jeonju Jeollabuk-do 561-232 (KR); RYU, Hyun-Seung, Yongin Gyeonggi-do 446-915 (KR); KIM, Giue-Nam, Seoul 135-090 (KR); KIM, Byoung-Suck, Seoul 150-942 (KR)
(74) Representative: Turner, Craig Robert
(86) International application number: PCT/KR2011/007518
(87) International publication number: WO 2013/047937

(57) **Abstract**

The present invention provides a composition for bone regeneration comprising: particulate demineralized bone matrix; fibrous demineralized bone matrix; and a hydrate. In addition, it is preferable that the present invention comprises a demineralized solution-derived isolated product obtained by neutralizing a demineralized solution, separated from a demineralization process, and separating the precipitate generated.

## Description

### TECHNICAL FIELD

The present invention relates to a bone-repair composition, more specifically to a bone-repair composition comprising a micro-particulate demineralized bone matrix in combination with a fibrous demineralized bone matrix or in combination with a fibrous demineralized bone matrix and a demineralizing solution-derived isolate.

### BACKGROUND ART

Demineralized bone matrix (DBM) refers to a bone whose minerals have been removed by adding it to an acid. Demineralized bone matrix mostly consists of highly cross-linked collagen and comprises the remaining non-collagenic proteins such as TGF-β, PDGF, osteopontin, osteonectin, bone morphogenetic protein (BMP) and the like. Demineralized bone matrix is used in a composition for bone implants, in the repair of bone defects, etc.

Demineralized bone matrix is obtained in a particulate form, through demineralizing a bone externally discharged from a body, followed by pulverizing into an appropriate size. Meanwhile, cortical bone consists of collagen fiber bundles that are oriented parallel to the long axis thereof. It is known that the fibrous demineralized bone matrix obtained therefrom exhibits properties useful for implants intended for use in bone repairs and other orthopedic applications. The fibrous demineralized bone matrix is prepared by milling a cortical bone externally discharged from a body with a special milling machinery until a particle having a fiber form is obtained, followed by demineralizing the resulting particles (US patent no. 5,607,269). However, there are some drawbacks, e.g., that such a method needs to use only intact cortical shafts as a bone source, because of the mechanical limitations of the bone milling machinery; and that the yield of the fibrous demineralized bone is very low. In order to address said problems, US patent nos. 7,323,193 and 7,939,108 have disclosed a process for preparing a fibrous demineralized bone matrix, comprising demineralizing the bone sections obtained from a bone externally discharged from a body in an acidic solution for 6 hours, demineralizing the resultant in an acidic solution for two days, and then pulverizing the demineralized bone sections. However, the processes disclosed in US patent nos. 7,323,193 and 7,939,108 have a drawback that the demineralizing step needs to be performed for long time, i.e., for two days (48 hours).

The present inventors have disclosed a bone-repair composition comprising a demineralized bone matrix having a particle size of 0.05 to 250 µm; a demineralized bone matrix having a particle size of 250 to 2000µm; and a hydrating material, wherein the demineralized bone matrixes are mixed in a certain ratio. The bone-repair composition has excellent injectability and shape-maintenance properties (Korean Patent No. 10-1041784). Although the bone-repair composition of Korean Patent No. 10-1041784 exhibits excellent properties in terms of injectability and shape-maintenance, there are drawbacks that it does not show satisfactory coherent strength due to low cohesive force; and that the application in the clinics causes adhering micro-materials to practitioner's hands (so called 'adhering phenomenon').

### DETAILED DESCRIPTION OF THE INVENTION

### TECHNICAL PROBLEM

The present inventors have performed various researches for developing bone-repair compositions which show excellent coherent strength and minimize adhering phenomenon, while still exhibiting excellent injectability and shape-maintenance. As a result, when bone-repair compositions are prepared using a fibrous demineralized bone matrix or a fibrous demineralized bone matrix and a demineralizing solution-derived isolate, instead of non-micropulverized demineralized bone matrix (i.e., a demineralized bone matrix having a particle size of 250 to 2000 µm) used in the present inventors' previous patent, it is found that the resulting composition shows both strong coherent strength and minimized adhering phenomenon, while maintaining injectability and shape-maintenance properties; and therefore that it is possible to prepare bone-repair compositions having excellent properties.

Therefore, it is an object of the present invention to provide a bone-repair composition comprising a micro-particulate demineralized bone matrix; a fibrous demineralized bone matrix, optionally a demineralizing solution-derived isolate, and a hydrating material.

### TECHNICAL SOLUTION

According to an aspect of the present invention, there is provided a bone-repair composition comprising a micro-particulate demineralized bone matrix; a fibrous demineralized bone matrix, and a hydrating material.

The micro-particulate demineralized bone matrix may have a particle size ranging from 0.05 to 250 µm. The fibrous demineralized bone matrix may have a length ranging from 1000 to 5000 µm.

The fibrous demineralized bone matrix may be obtained by a process comprising (a) performing a first demineralizing step by demineralizing a bone externally discharged from a body in an acidic solution for 1 to 5 hours; (b) slicing the bone obtained from the step (a) to form a bone in a sheet form having a thickness of 0.1 to 3 mm; (c) performing a second demineralizing step by demineralizing the bone in a sheet form obtained from the step (b) in an acidic solution for 2 to 6 hours; and (d) pulverizing the demineralized bone obtained from the step (c). The acidic solutions in the steps (a) and (c) may be, independently each other, a 0.1 to 3 N HCl solution, preferably a about 0.6 N HCl solution.

The hydrating material may be one or more selected from the group consisting of distilled water, saline, concentrated saline, and ion solution.

The weight ratio of the micro-particulate demineralized bone matrix : the fibrous demineralized bone matrix : the hydrating material may be 1 : 0.3 to 1.2 : 3 to 10.

And also, the bone-repair composition of the present invention may further comprise a demineralizing solution-derived isolate obtained by neutralizing a demineralizing solution recovered after performing demineralization and then isolating the resulting precipitate. The demineralizing solution-derived isolate may be present in an amount ranging from 1 to 5 parts by weight, based on 1 part by weight of the micro-particulate demineralized bone matrix.

### ADVANTAGEOUS EFFECTS

The bone-repair composition of the present invention comprises a micro-particulate demineralized bone matrix in combination with a fibrous demineralized bone matrix or in combination with a fibrous demineralized bone matrix and a demineralizing solution-derived isolate. The bone-repair composition of the present invention can remarkably increase coherent strength and effectively minimize adhering phenomenon, while maintaining injectability and shape-maintenance properties equal to the bone-repair composition previously developed by the present inventors. Therefore, the bone-repair composition according to the present invention has excellent properties, thereby being able to be usefully applied to implants for the purpose of the repair of bone defects, etc.

### BRIEF DESCRIPTION OF DRAWINGS

FIG. 1 shows the bones obtained by slicing a partially demineralized bone (A) and the bones obtained by pulverizing a demineralized bone (B).
FIG. 2 shows pictures obtained by observing with an optical microscope the demineralized bone matrix in a fiber form (A) and the demineralized bone matrix in a particulate form (B).
FIGs. 3 and 4 show the picture obtained by observing the demineralizing solution-derived isolate with an optical microscope; and the particle size distribution of the demineralizing solution-derived isolate, respectively.
FIGs. 5 and 6 show the results of TGA and XRD analyses on the demineralizing solution-derived isolate.

### BEST MODE FOR CARRYING OUT THE INVENTION

The present invention provides a bone-repair composition comprising a micro-particulate demineralized bone matrix; a fibrous demineralized bone matrix, and a hydrating material.

The bone-repair composition according to the present invention can remarkably increase coherent strength and effectively minimize adhering phenomenon, while maintaining injectability and shape-maintenance properties equal to the known bone-repair composition (specifically, the bone-repair composition according to Korean Patent No. 10-1041784). Therefore, the bone-repair composition according to the present invention has excellent properties, thereby being able to be usefully applied to implants for the purpose of the repair of bone defects, etc.

The micro-particulate demineralized bone matrix may be prepared, according to conventional methods for preparing demineralized bone matrix, using the micro-pulverized bone obtained by micro-pulverizing a bone externally discharged from a body. The bone externally discharged from a body may be a bone derived from a mammal, including a human. It is preferable to use the bone obtained after removing soft tissues, lipids, bone marrow, etc. from the bone externally discharged from a body, according to conventional methods. The removing may be carried out using e.g., 60 to 90 wt/wt% ethanol solution. If necessary, a surfactant may be additionally used. The demineralizing may be performed according to conventional demineralizing methods, for example by placing a micro-pulverized bone in a 0.6 N HCl solution for about 1 to 6 hours. Finally, the resulting micro-particulate demineralized bone matrix may be obtained through neutralizing, washing, and lyophilizing. The micro-particulate demineralized bone matrix having a particle size of 250 µm or less, preferably a particle size ranging from 0.05 to 250 µm, may be used without limitation.

The fibrous demineralized bone matrix, which is a demineralized bone matrix having a fiber form, may have a length ranging from 1000 to 5000 µm, preferably from 2,000 to 4,000 µm. The fibrous demineralized bone matrix may be prepared according to conventional methods, for example according to the methods disclosed in US patent nos. 7,323,193 and 7,939,108. Meanwhile, the present inventors have found that, through performing sequential steps for partial demineralizing, slicing, complete demineralizing, and pulverizing, the demineralizing step can be remarkably reduced to about 6 hours; and the resulting demineralized bone matrix can be obtained in high yield as a demineralized bone matrix having a fiber form. Therefore, the fibrous demineralized bone matrix may be obtained by a process comprising: (a) performing a first demineralizing step by demineralizing a bone externally discharged from a body in an acidic solution for 1 to 5 hours; (b) slicing the bone obtained from the step (a) to form a bone in a sheet form having a thickness of 0.1 to 3 mm; (c) performing a second demineralizing step by demineralizing the bone in a sheet form obtained from the step (b) in an acidic solution for 2 to 6 hours; and (d) pulverizing the demineralized bone obtained from the step (c).

The bone externally discharged from a body used in the step (a) may be a bone derived from a mammal, including a human. It is preferable to use the bone obtained after removing soft tissues, lipids, bone marrow, etc. from the bone externally discharged from a body, according to conventional methods. The removing may be carried out using e.g., 60 to 90 wt/wt% ethanol solution. If necessary, a surfactant may be additionally used. Typically, the acidic solution may be a HCl solution such as a 0.1 to 3 N HCl solution, preferably about a 0.6 N HCl solution. And also, the first demineralizing step may be performed for 1 to 5 hours, preferably about 3 hours.

The slicing in the step (b) may be performed with an appropriate apparatus for thinly cutting a bone, for example with a bone slicer such as Bone Slicer (YOU IL MC/CO. KR), but not limited thereto. The thickness of the bone in a sheet form obtained by said slicing may range from 0.1 to 3 mm, preferably from 0.2 to 1.0 mm, more preferably from 0.3 to 0.6 mm, most preferably about 0.5 mm. When the thickness exceeds 3.0 mm, broken particulate forms (not a sheet form) may be obtained. When the thickness is below 0.1 mm, a sheet form can be obtained; but subsequent demineralization of the resulting sheet form may give particulate forms (not a fiber form).

Typically, the acidic solution in the step (c) may be a HCl solution such as a 0.1 to 3 N HCl solution, preferably about a 0.6 N HCl solution. The second demineralizing step, i.e., complete demineralizing step, can be performed in a remarkably reduced time (i.e., in 2 to 6 hours, preferable in about 3 hours), in comparison with known demineralizing methods.

The pulverizing in the step (d) may be performed with a conventional pulverizing apparatus. The pulverizing may be carried out so as to obtain a fibrous demineralized bone matrix having a length ranging from 1000 to 5000 µm, preferably from 2,000 to 4,000 µm, which can be accomplished by setting appropriate pulverizing conditions according to types of the pulverizing apparatus used.

The hydrating material may be one or more selected from the group consisting of distilled water, saline, concentrated saline, and ion solution.

The weight ratio of the micro-particulate demineralized bone matrix : the fibrous demineralized bone matrix : the hydrating material may be 1 : 0.3 to 1.2 : 3 to 10, preferably 1 : 0.6 to 0.8 : 3 - 10.

And also, the bone-repair composition of the present invention may further comprise a demineralizing solution-derived isolate obtained by neutralizing a demineralizing solution recovered after performing demineralization and then isolating the resulting precipitate. The demineralizing solution-derived isolate may be present in an amount ranging from 1 to 5 parts by weight, preferably 2 to 4 parts by weight, based on 1 part by weight of the micro-particulate demineralized bone matrix. As used herein, the term "demineralizing solution" refers to a solution recovered after performing demineralization, which is usually separated and discarded as waste liquid. The neutralizing may be carried out using a NaOH solution, preferably in two steps using NaOH solutions. For example, the neutralizing may be carried out by adjusting to the pH 4 with a NaOH solution, standing for 10 minutes, and then adjusting to about pH 7. The resulting precipitate after the neutralization may be isolated according to conventional methods, e.g., filtration, centrifugation, etc. It is found that the demineralizing solution-derived isolate mainly consists of particles having a particle size of 6 to 70 µm, which contain about 87 wt/wt% of inorganic materials and about 5 wt/wt% of organic materials. It is also found that the inorganic materials are mainly amorphous hydroxyapatite.

The bone-repair composition of the present invention may contain a liquid polyhydroxy compound, examples of which include glycerol or glycerol ester.

The bone-repair composition of the present invention may also contain a biocompatible binder. Examples of the biocompatible binder include one or more selected from the group consisting of fibrin adhesive, fibrinogen, thrombin, mussel adhesive protein, silk, elastin, collagen, casein, gelatin, albumin, keratin, chitin and chitosan. Other examples of the biocompatible binder include one or more selected from the group consisting of starch, polylactic acid, polyglycolic acid, polylactic-co-glycolic acid, polydioxanone, polycaprolactone, polycarbonate, polyoxoester, polyamino acid, poly-anhydride, polyhydroxybutylate, polyhydroxyvalerate, poly(propylene glycol-co-fumaric acid), tyrosine-based-polycarbonate, polyvinylpyrrolidone, cellulose, ethyl cellulose and carboxymethyl cellulose.

The bone-repair composition of the present invention may also contain one or more selected from the group consisting of antibiotic, vitamins, glucosamine, cytokine and growth factors.

The bone-repair composition of the present invention may further contain an auxiliary component that can help bone repair. Any commercially available auxiliary components that can help bone repair can be used for the composition of the present invention. Especially, one or more selected from the group consisting of cancellous bone chips, compact bone chips, hydroxyapatite (HA, Ca₁₀(PO₄)₆(OH)₂), carbonic acid apatite (CA, Ca₁₀(PO₄)₆CO₃), tricalcium phosphate (TCP, Ca₃(PO₄)₂), calcium pyrophosphate (Ca₂P₂O₇), anorganic bone, dental tooth enamel, aragonite, calcite, nacre, graphite, pyrolytic carbon, calcium-silicate-based bioglass, alumina (Al₂O₃) and zirconia (ZrO₂) are preferable.

The present invention will be described in further detail with reference to the following examples. However, these examples are for illustrative purposes only and are not intended to limit the scope of the present invention.

### Example 1

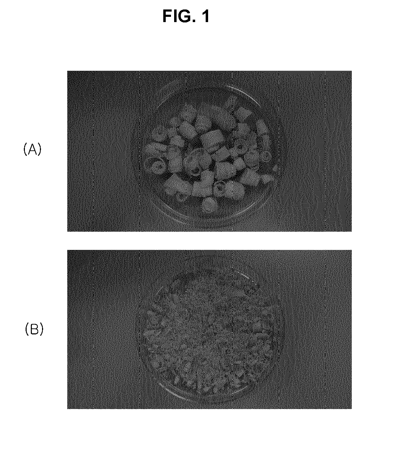

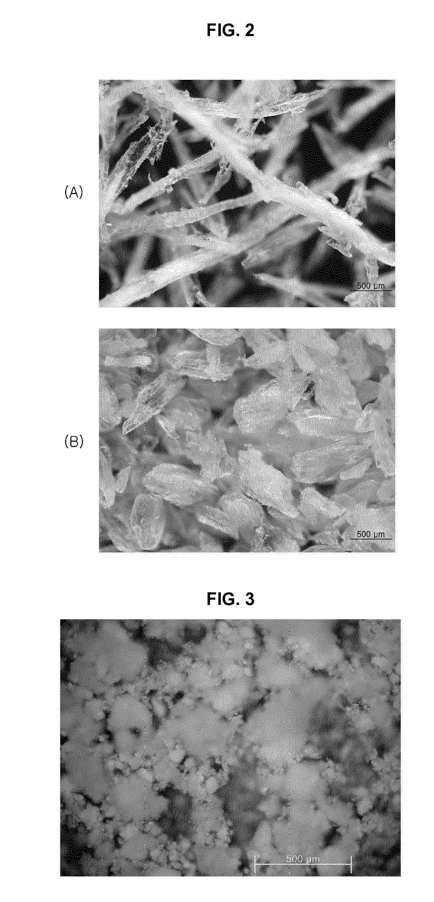

From a bone (weight: about 172 g) of human donor origin, soft tissues attached to the bone were removed with a surgical instrument; and then impurities such as soft tissues, lipids, and bone marrow were removed using a tissue detergent containing a surfactant. The resulting bone was cut into a half. The cut bones were placed in 20 ml of a 0.6N HCl solution per 1 g of the bone for 3 hours, for partial demineralization. The partially demineralized bones were isolated and then placed in 20 ml of distilled water per 1 g of the bone, so as to remove the demineralizing solution in the bones. The resulting bones were sliced with a bone slicer (YOU IL MC/CO. KR) into a sheet form having a thickness of about 0.5 mm. The resulting bones in a sheet form are shown in FIG 1 (A). The resulting bones in a sheet form were placed in 30 ml of a 0.6N HCl solution per 1 g of the bone for 3 hours, for complete demineralization. The precipitated demineralized bone matrix was isolated, pulverized with a pulverizing apparatus (IKA, M20 Universal mill, GR) for about 10 minutes, neutralized with phosphate buffered saline (PBS), washed with distilled water, and then lyophilized to obtain about 31 g of the demineralized bone matrix. The picture obtained by observing the resulting demineralized bone matrix with an optical microscope is shown in FIG 2(A). From the result of FIG 2 (A), it can be seen that the resulting demineralized bone matrix is a demineralized bone matrix having a fiber form.

### Comparative Example 1

From a bone (weight: about 165 g) of human donor origin, soft tissues attached to the bone were removed with a surgical instrument; and then impurities such as soft tissues, lipids, and bone marrow were removed using a tissue detergent containing a surfactant. The resulting bone was cut into a half. The cut bones were placed in 20 ml of a 0.6N HCl solution per 1 g of the bone for 3 hours, for partial demineralization. The partially demineralized bones were isolated and then placed in 20 ml of distilled water per 1 g of the bone, so as to remove the demineralizing solution in the bones. The resulting bones were again placed in 30 ml of a 0.6N HCl solution per 1 g of the bone for 3 hours, for demineralization. However, because the demineralization was hardly processed, we could not obtain a demineralized bone matrix.

### Comparative Example 2

From a bone (weight: about 192 g) of human donor origin, soft tissues attached to the bone were removed with a surgical instrument; and then impurities such as soft tissues, lipids, and bone marrow were removed using a tissue detergent containing a surfactant. The resulting bone was cut into a half. The cut bones were placed in 20 ml of a 0.6N HCl solution per 1 g of the bone for 3 hours, for partial demineralization. The partially demineralized bones were isolated and then placed in 20 ml of distilled water per 1 g of the bone, so as to remove the demineralizing solution in the bones. The resulting bones were pulverized with a pulverizing apparatus (IKA, M20 Universal mill, GR) for about 60 minutes to obtain pulverized bones, which are shown in FIG 1 (B). The pulverized bones were placed in 30 ml of a 0.6N HCl solution per 1 g of the bone for 3 hours, for complete demineralization. The precipitated demineralized bone matrix was isolated, neutralized with phosphate buffered saline (PBS), washed with distilled water, and then lyophilized to obtain about 36.4 g of the demineralized bone matrix. The picture obtained by observing the resulting demineralized bone matrix with an optical microscope is shown in FIG 2 (B). From the result of FIG 2 (B), it can be seen that the resulting demineralized bone matrix is a demineralized bone matrix having a particulate form.

### Example 2

### (1) Preparation of micro-particulate demineralized bone matrix

From a bone (weight: about 245 g) of human donor origin, soft tissues attached to the bone were removed with a surgical instrument; and then impurities such as soft tissues, lipids, and bone marrow were removed using a tissue detergent containing a surfactant. The resulting bone was cut to the sizes sufficient for putting into a pulverizing apparatus. The cut bones were pulverized to the particle size of 250 µm or less with a pulverizing apparatus (IKA, M20 Universal mill, GR). The pulverized bone particles were placed in a 0.6N HCl solution for 3 hours, for complete demineralization. The demineralized bone matrix was isolated by centrifugation and the resulting demineralizing solution was stored separately. The demineralized bone matrix was neutralized with phosphate buffered saline (PBS), washed with distilled water, and then lyophilized to obtain about 53.1 g of the micro-particulate demineralized bone matrix.

### (2) Preparation of demineralizing solution-derived isolate and evaluation thereof

### (2-1) Preparation of demineralizing solution-derived isolate

The demineralizing solutions recovered from Example 1 and the above (1) were combined in a vessel. The pH of the solution was adjusted to about pH 4 with a 2N NaOH solution. After standing the solution for 10 minutes, the pH of the solution was adjusted to pH 7.2 with a 2N NaOH solution. The solution was centrifuged at 4,000 rpm to isolate the precipitate, which was then lyophilized to prepare a demineralizing solution-derived isolate.

### (2-2) Property evaluation of demineralizing solution-derived isolate

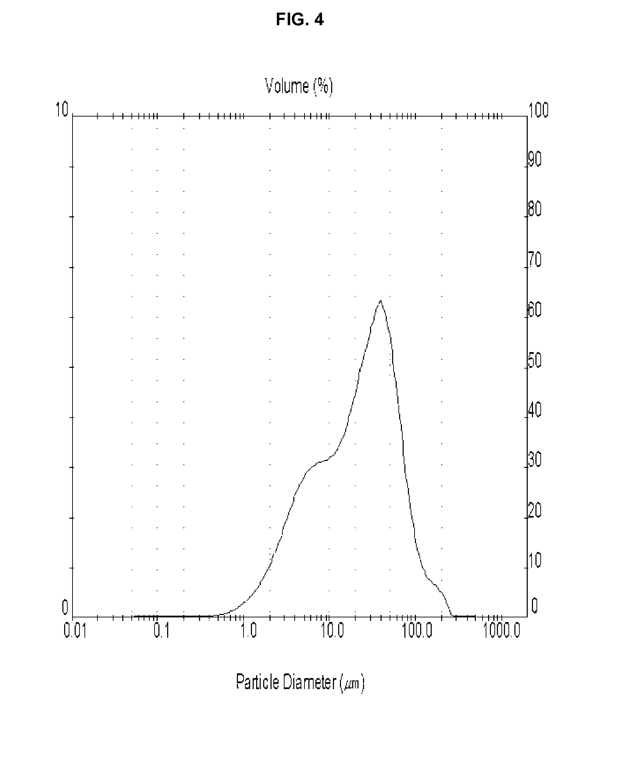

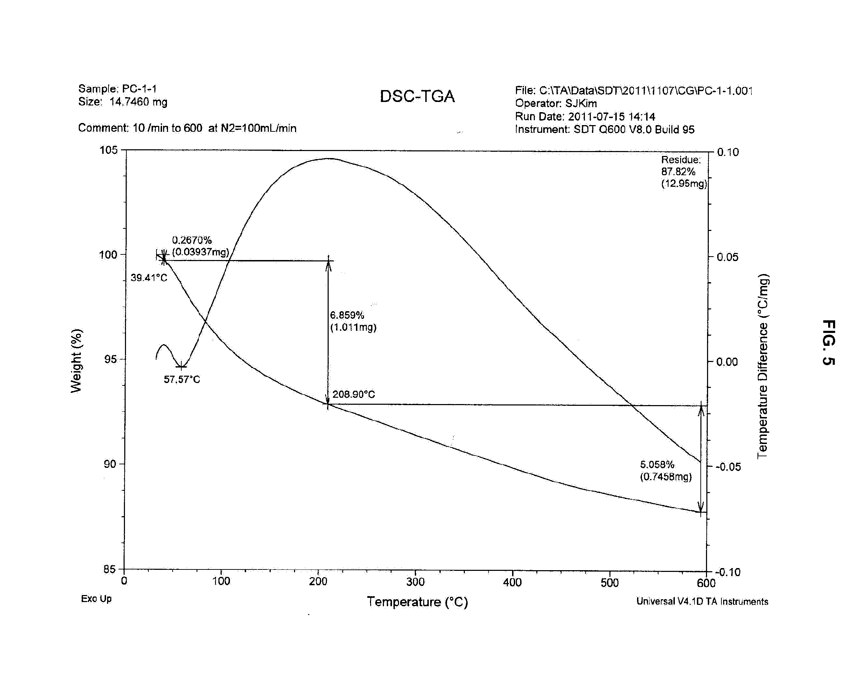

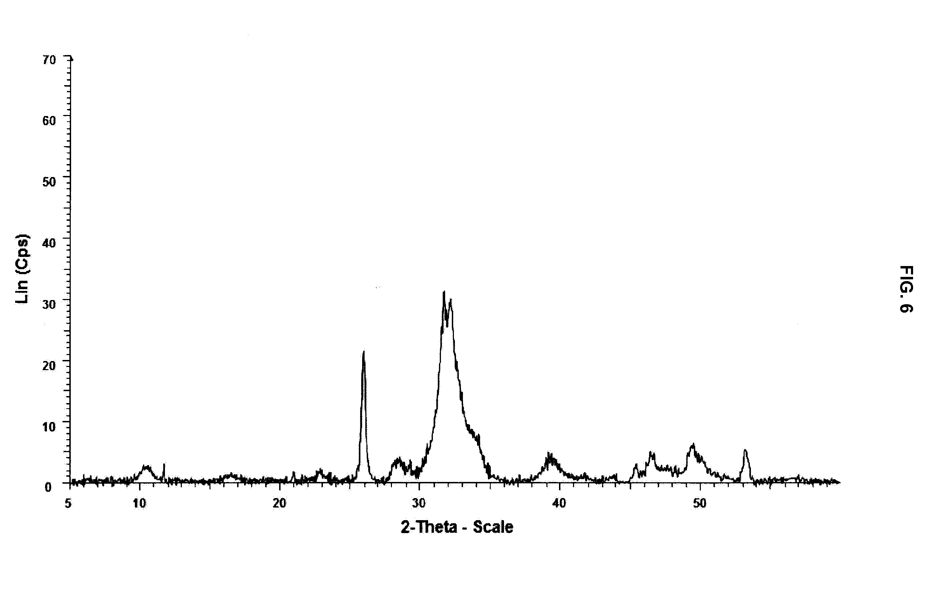

The result obtained by observing the appearance of the demineralizing solution-derived isolate prepared in the above (2-1) with an optical microscope is shown in FIG 3. The particle size distribution thereof is shown in FIG 4. It can be seen that the demineralizing solution-derived isolate mainly consists of particles having a particle size of 6 to 70 µm. And also, the results of TGA and XRD analyses on the demineralizing solution-derived isolate are shown in FIG 5 and FIG 6, respectively. As shown in the result of FIG 5, the demineralizing solution-derived isolate contains about 87 wt/wt% of inorganic materials and about 5 wt/wt% of organic materials. In addition, as shown in the result of FIG 6, the inorganic materials are mainly amorphous hydroxyapatite. In the XRD result (i.e., FIG 6), the 2-theta intervals of 20 to 25 and 25 to 30 mainly show amorphous hydroxyapatite.

### Example 3: Preparation of bone-repair composition and evaluation thereof

The fibrous demineralized bone matrix prepared in Example 1, the micro-particulate demineralized bone matrix prepared in Example 2, the demineralizing solution-derived isolate, and distilled water were mixed according to the weight ratios shown in Table 2 below; and then the properties were evaluated. For comparison, we also evaluated the properties of the bone-repair composition which was prepared with the particulate demineralized bone matrix having a particle size of 250~750 µm.

Ten samples of each bone-repair composition were prepared. Each composition was put into a syringe in the same amount, and the piston was pushed into the cylinder by finger to extrude the composition. Injectability was determined considering ease of extrusion and the shape of the extruded composition. In addition, shape-maintenance and coherent strength, as well as adherence to hand, were determined after massing the extruded composition by hand. Each property, i.e., injectability (A), shape-maintenance (B), coherent strength (C), and adherence to hand were evaluated in 5 levels as indicated in Table 1 below, and the results are shown in Table 2 below.

**Table 1**

| Point | Injectability (A) | Shape-maintenance (B) | Coherent strength (C) | Adherence to hand (D) |
|---|---|---|---|---|
| 4 | easily injected and extruded with no cutting | shapable with no break or crack | strength shown along with strong cohesive force | almost not adhered |
| 3 | easily injected but partially cut and cracked | can be massed but partially broken or cracked when shaped | moderate cohesive force shown | small materials slightly adhered |
| 2 | extruded in broken state | cannot be massed when broken | week cohesive force shown and soft | lots of small materials adhered |
| 1 | extruded but run | cannot be massed | cannot be massed | lots of both small materials and fibers adhered |
| 0 | extruded in powder state | run | run | run |

**Table 2**

| Test group | Particulate demineralized bone matrix | | Fibrous demineraliz ed bone matrix | Demineral izing solution-derived isolate | Distilled water | (A) | (B) | (C) | (D) |
|---|---|---|---|---|---|---|---|---|---|
| | 250 µm or less | 250-750 µm | | | | | | | |
| Group 1 | 1 | 0 | 0.7 | 0 | 3.3 | 4 | 4 | 3 | 3 |
| Group 2 | 1 | 0 | 0.7 | 0.5 | 2.25 | 4 | 4 | 4 | 1 |
| Group 3 | 1 | 0 | 0.7 | 1.0 | 3.0 | 4 | 4 | 4 | 2 |
| Group 4 | 1 | 0 | 0.7 | 1.5 | 3.25 | 4 | 3 | 3 | 2 |
| Group 5 | 1 | 0 | 0.7 | 2.0 | 3.3 | 4 | 4 | 4 | 4 |
| Group 6 | 1 | 0 | 0.7 | 2.5 | 3.6 | 4 | 4 | 4 | 3 |
| Group 7 | 1 | 0 | 0.7 | 5.0 | 3.85 | 4 | 4 | 4 | 2 |
| Group 8 | 1 | 0 | 0.7 | 4.0 | 4.15 | 4 | 4 | 4 | 2 |
| Group 9 | 1 | 0 | 0.7 | 5.0 | 4.5 | 4 | 4 | 4 | 2 |
| Comparative group 1 | 0.8 | 1 | 0 | 0 | 3.8 | 4 | 4 | 2 | 2 |
| Comparative group 2 | 0 | 0 | 0.7 | 2 | 3.3 | 2 | 1 | 1 | 1 |
| Comparative group 3 | 1 | 0 | 0 | 2 | 3.3 | 3 | 3 | 2 | 2 |
| Comparative group 4 | 1 | 0 | 0.7 | 0 | 0 | 0 | 0 | 0 | 0 |

As shown in Table 2 above, it can be seen that, when the particulate demineralized bone matrix having a particle size of 250 to 750 µm is changed to the fibrous demineralized bone matrix, coherent strength is increased and adhering phenomenon is decreased, while still maintaining injectability and shape-maintenance (see Test group 1 vs. Comparative group 1). It is presumed that these results are derived from the facts that the micro-particulate demineralized bone matrix fills the interspaces of the fibrous demineralized bone matrix, thereby increasing injectability and shape-maintenance of the composition; and that coherence of the fibrous demineralized bone matrix *per se* leads to increased coherent strength and decreased adhering phenomenon.

And also, when the demineralizing solution-derived isolate was additionally added, the increase of coherent strength and the decrease of adhering phenomenon became more distinct. Especially, the addition of 2.0 parts by weight of the demineralizing solution-derived isolate based on 1 part by weight of the micro-particulate demineralized bone matrix showed more excellent properties (see Test groups 1 to 9). And also, the compositions containing only one of the micro-particulate demineralized bone matrix and the fibrous demineralized bone matrix, along with the demineralizing solution-derived isolate, did not show improved properties (see Comparative groups 2 and 3). Therefore, from the above results, it can be seen that the composition needs to contain both the micro-particulate demineralized bone matrix and the fibrous demineralized bone matrix in an appropriate ratio, preferably along with 2.0 parts by weight of the demineralizing solution-derived isolate based on 1 part by weight of the micro-particulate demineralized bone matrix.

## Claims

1. A bone-repair composition comprising a micro-particulate demineralized bone matrix; a fibrous demineralized bone matrix, and a hydrating material.

2. The bone-repair composition of claim 1, wherein the micro-particulate demineralized bone matrix has a particle size ranging from 0.05 to 250 µm.

3. The bone-repair composition of claim 1, wherein the fibrous demineralized bone matrix has a length ranging from 1000 to 5000 µm.

4. The bone-repair composition of claim 1, wherein the fibrous demineralized bone matrix obtained by a process comprising (a) performing a first demineralizing step by demineralizing a bone externally discharged from a body in an acidic solution for 1 to 5 hours; (b) slicing the bone obtained from the step (a) to form a bone in a sheet form having a thickness of 0.1 to 3 mm; (c) performing a second demineralizing step by demineralizing the bone in a sheet form obtained from the step (b) in an acidic solution for 2 to 6 hours; and (d) pulverizing the demineralized bone obtained from the step (c).

5. The bone-repair composition of claim 4, wherein the acidic solutions in the steps (a) and (c) are, independently each other, a 0.1 to 3 N HCl solution.

6. The bone-repair composition of claim 5, wherein the acidic solutions in the steps (a) and (c) are a 0.6 N HCl solution.

7. The bone-repair composition of claim 1, wherein the hydrating material is one or more selected from the group consisting of distilled water, saline, concentrated saline, and ion solution.

8. The bone-repair composition of claim 1, wherein the weight ratio of the micro-particulate demineralized bone matrix : the fibrous demineralized bone matrix : the hydrating material is 1 : 0.3 to 1.2 : 3 to 10.

9. The bone-repair composition of any one of claims 1 through 8, further comprising a demineralizing solution-derived isolate obtained by neutralizing a demineralizing solution recovered after performing demineralization and then isolating the resulting precipitate.

10. The bone-repair composition of claim 9, wherein the demineralizing solution-derived isolate is present in an amount ranging from 1 to 5 parts by weight, based on 1 part by weight of the micro-particulate demineralized bone matrix.
